# EUROPEAN PATENT APPLICATION

(11) **EP 0 175 180 A1**
(43) Date of publication of application: **26.03.1986**
(21) Application number: 85110756.5
(22) Date of filing: 27.08.1985
(51) Int. Cl.: C07D 233/64, A61K 31/415

(54) **Histargin-related compounds, a process for their preparation and their use as medicaments**

(30) Priority: 05.09.1984 JP 184384/84
(71) Applicant: MICROBIAL CHEMISTRY RESEARCH FOUNDATION, Tokyo 141 (JP)
(72) Inventor: Umezawa, Hamao, Prof., Nerima-ku Tokyo (JP); Takeuchi, Tomio, Shinagawa-ku Tokyo (JP); Aoyagi, Takaaki, Fujisawa-city Kanagawa Prefecture (JP); Iinuma, Hironobu, Wako-city Saitama Prefecture (JP); Ogawa, Keiji, Hachioji-city Tokyo (JP); Moriguchi, Makoto, Joyo-city Kyoto Prefecture (JP); Umeda, Yoshihisa, Ohtsu-city Shiga Prefecture (JP); Kuroda, Hiroyuki, Koga-gun Shiga Prefecture (JP); Nakamura, Teruya, Kusatsu-City Shiga Prefecture (JP); Ohbayashi, Akira, Uji-city Kyoto Prefecture (JP)
(74) Representative: Becker, Heinrich Karl Engelbert, Dr.

(57) **Abstract**

The present invention relates to compounds of the formula 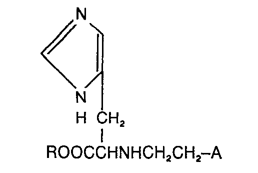 wherein A is a residual group obtained by removing one hydrogen atom from the alpha-amino group in an alpha-amino acid (excluding arginine) or a lower alkyl ester thereof; R is a hydrogen atom or a lower alkyl group, or a salt thereof, and to a process for the preparation of these compounds.

The compounds are capable of inhibiting the action of angiotensin converting enzyme and can therefore be used as anti-hypertensive agents.

## Description

The present invention relates to novel Histargin related compounds capable of inhibiting the action of angiotensin converting enzyme.

Histargin is a compound isolated from the filtrate of a cultured broth of Histargin-producing Streptomyces roseoviridis MF 118-A5 (deposited under FERM-P No. 7043). Histargin has the following chemical structure:

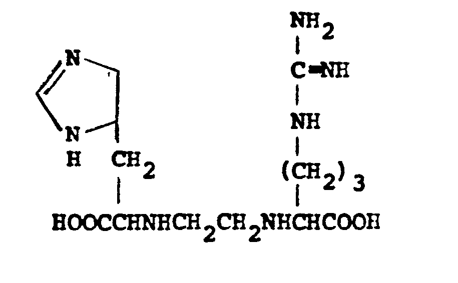

Histargin has a carboxypeptidase B inhibition activity and can, therefore, be used as an analgesic or potentiation of analgesic effect because of its ability to inhibit the action of enkephalinase (see Japanese Patent Application No. 89075/1983).

Histargin is a new type of compound wherein the alpha-amino groups in histidine and arginine are inter- bonded by an ethylene group, and physiologically active substances having a stronger inhibiting action could be found by screening Histargin analogues having the two amino acids converted to other amino acids. Histargin analogues are also suitable for use in searching for compounds having the ability to inhibit peptidases other than carboxypeptidase B and enkephalinase. The principal object of the present invention is to present a group of such Histargin related compounds.

The present invention relates to Histargin related compounds of the following formula (I), or salts thereof: 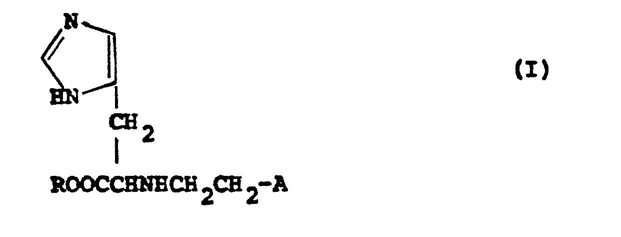 (wherein A is a residual group obtained by removing one hydrogen atom from the alpha-amino group in an alpha-amino acid (excluding arginine) or a lower alkyl ester thereof; R is a hydrogen atom or a lower alkyl group).

As a result of various studies on Histargin related compounds wherein the two amino acids (histidine and arginine) in Histargin are converted to various other amino acids or esters thereof, it has been found that the novel Histargin related compounds of formula (I) wherein the arginine portion in Histargin is converted to other alpha-amino acids have a great ability to inhibit the action of angiotensin converting enzyme. The present invention has been accomplished on the basis of this finding. The compounds in accordance with the present invention can be used as hypotensives. Part of such compounds have the ability to inhibit the action of carboxypeptidase B and/or carboxypeptidase A and can be used as reagents in biochemical studies on these enzymes.

The symbol A in formula (I) for the compounds in accordance with the present invention represents a residual group obtained by removing one hydrogen atom from the alpha-amino group in an alpha-amino acid (excluding arginine) or a lower alkyl ester thereof (said residual group is hereunder simply referred to as the alpha-amino acid residue). Any such residual group that is derived from known alpha-amino acids may be used as A. If the alpha-amino acid residue has an optically active carbon atom, the residue may be any of the L-, D- or DL-type. The histidine portion in formula (I) may also be any of these three isomer types. Illustrative alpha-amino acids are listed below: glycine, alanine, alpha-aminobutyric acid, proline, valine, norvaline, isoleucine, alloisoleucine, leucine, norleucine, serine, homoserine, threonine, allothreonine, o-methylserine, o-ethylserine, o-methylhomoserine, o-ethylhomoserine, o-methylthreonine, o-ethylthreonine, o-methylallothreonine, o-ethylallothreonine, ornithine, lysine, aspartic acid, glutamic acid, asparagine, glutamine, phenylalanine, tyrosine, histidine, tryptophane, cysteine, homocysteine, s-methylcysteine, s-ethylcysteine, methionine and ethionine.

The Histargin related compounds in accordance with the present invention may be handled either as intramolecular salts or pharmaceutically acceptable salts thereof. Illustrative salts include those with pharmaceutically acceptable cations at the carboxyl group such as sodium ion, potassium ion, calcium ion and magnesium ion, as well as pharmaceutically acceptable inorganic and organic salts at the imino group, imidazolyl group or the amino group at the side chain in the alpha-amino acid.

As will be shown later in the Examples, the synthesis of most of the Histargin related compounds in accordance with the present invention includes as the final steps the removal of various protective groups and purification with an ion-exchange resin such as Dowex @ 50W (product of Dow Chemical Co.). Non-crystalline compounds may be obtained as a white powder by freeze-drying, whereas crystalline compounds are obtained as a colorless crystal by recrystallization.

For the sake of convenience in the following description, the compound of formula (I) is represented by the abbreviations for the two constituent amino acids connected by -C₂-. For example, His-C₂-Phe represents a compound wherein the alpha-amino groups in histidine and phenylalanine are bonded by an ethylene group. A protected Histargin related compound which is used as an intermediate for the synthesis of the compound of formula (I) is represented by the abbreviations for the two protected amino acids connected by -C₂-. For example, Z-L-His-C₂-L-Lys(Z)-OMe represents a compound wherein the alpha-amino groups in N^{α-}benzyloxycarbonyl-L-histidine and N^{E-}benzyloxycarbonyl-L-lysine methyl ester are bonded by an ethylene group.

Typical examples of the compound in accordance with the present invention are listed in Table 1 together with their structures, abbreviations and molecular formulas. The IR absorption spectra (KBr tab.) and proton NMR spectra [reference: sodium 2,2-dimethyl-2-silapentane-5-sulfonate (DSS) in D₂0] of the compounds wherein both amino acids are in the L-form are shown in Table 2. The specific rotations and melting points of these compounds are listed in Table 3.

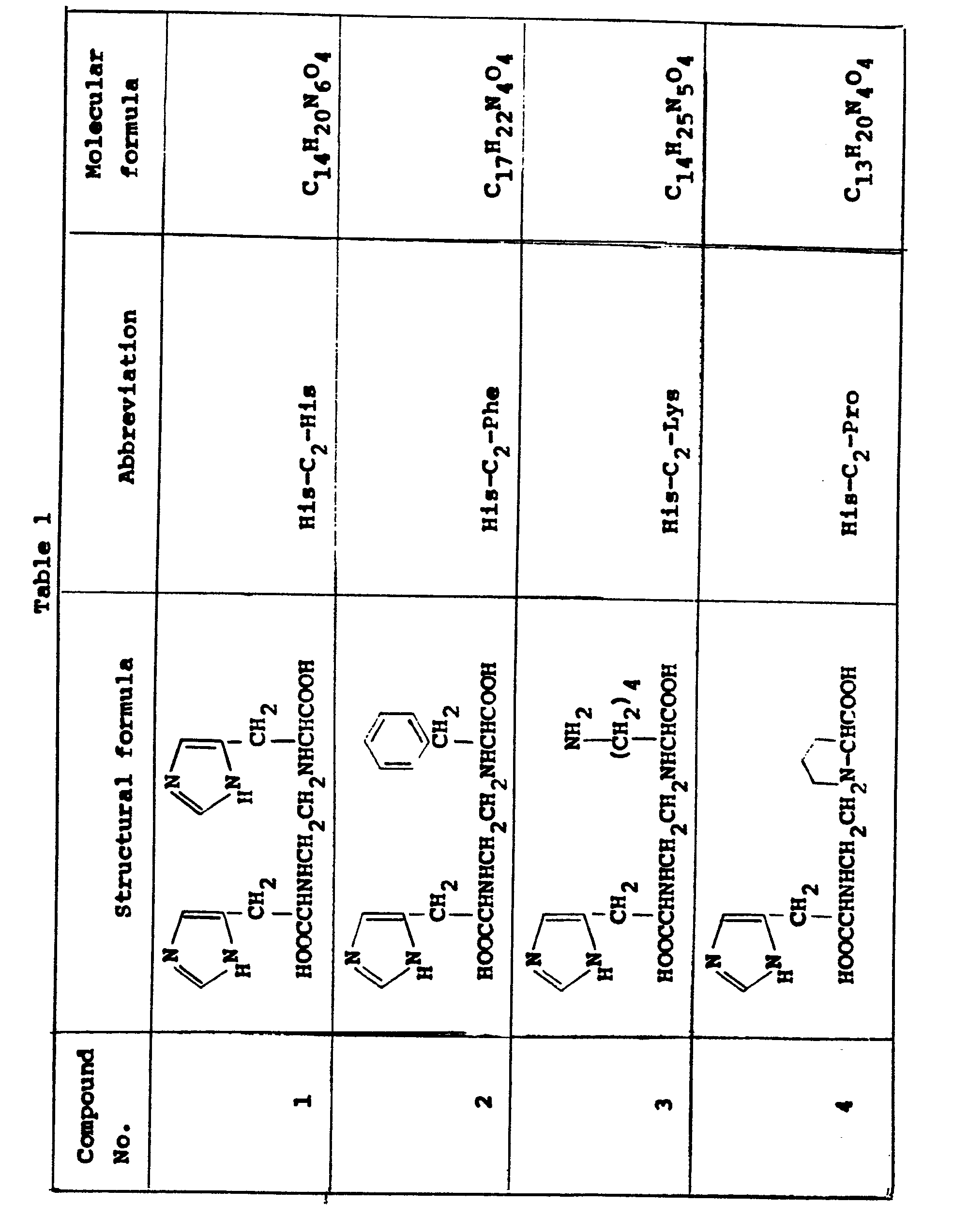

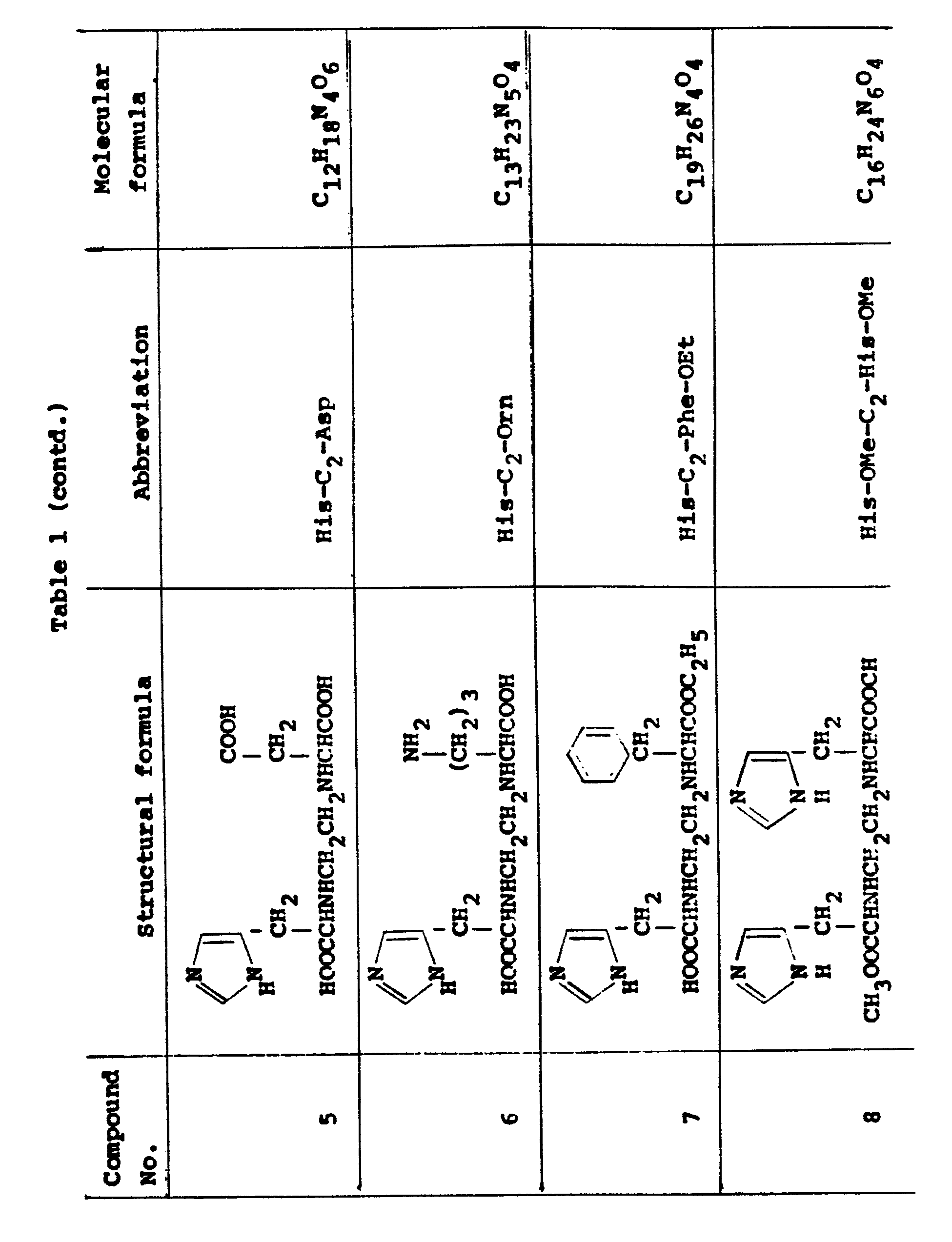

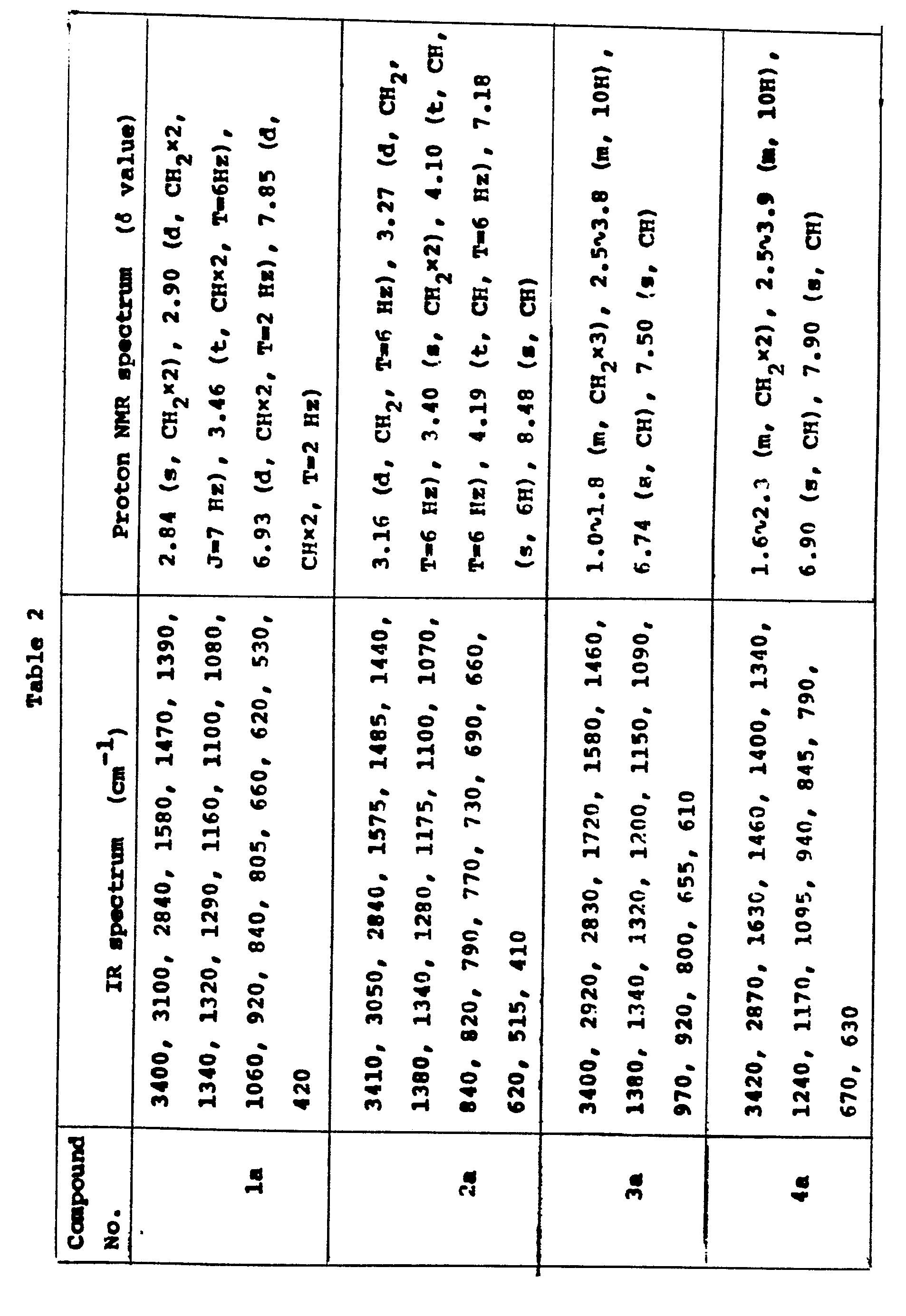

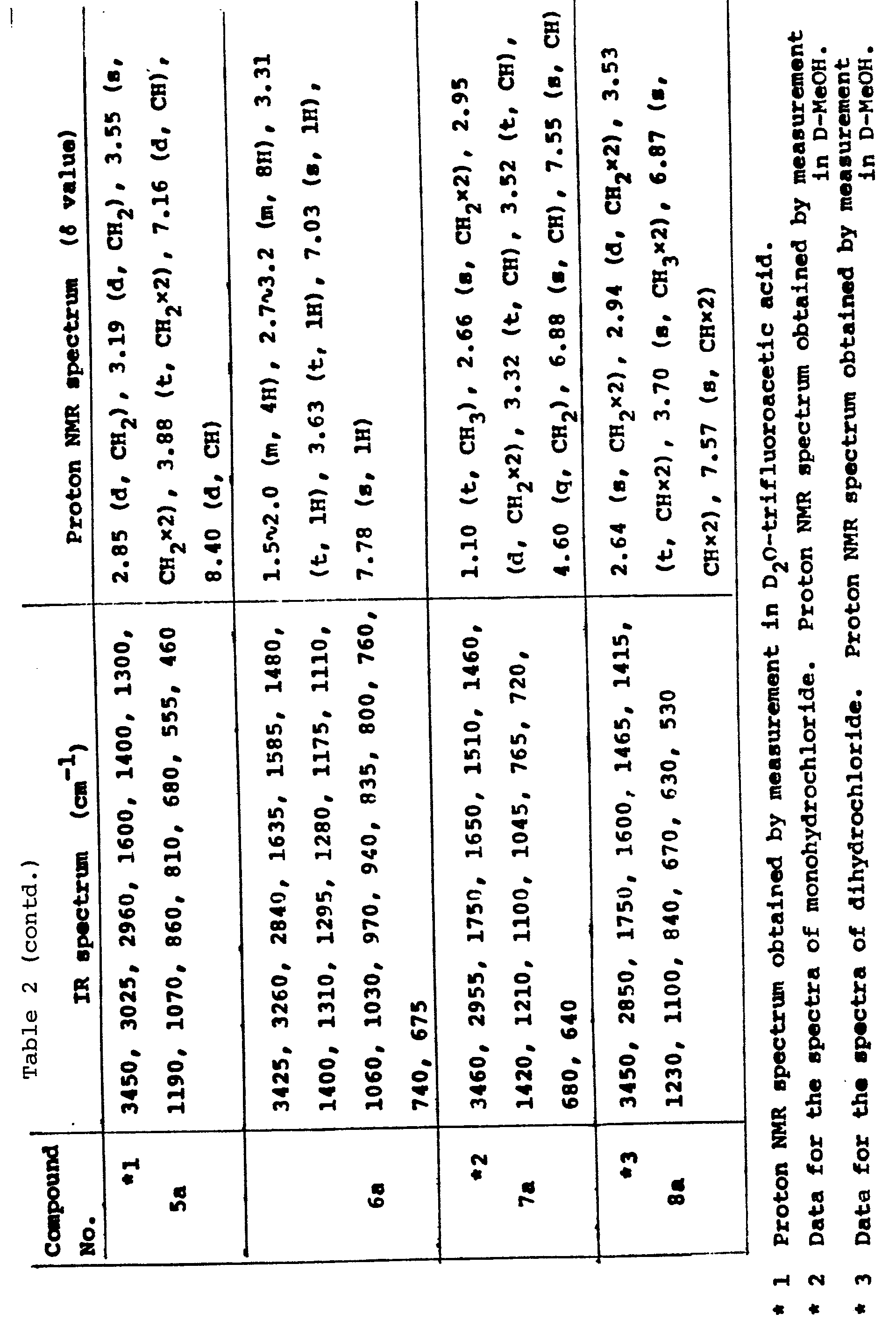

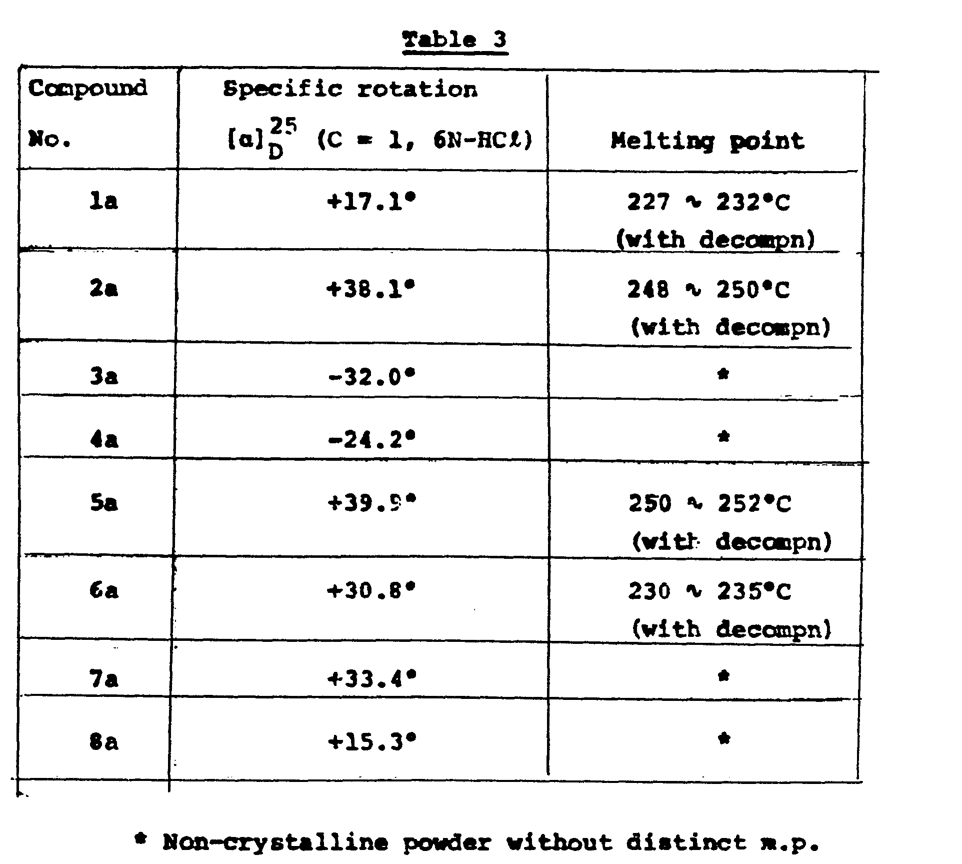

All of the compounds in accordance with the present invention exhibit a great ability to inhibit the action of angiotensin converting enzyme. Part of the compounds also exhibit the ability to inhibit the action of carboxypeptidase B and/or carboxypeptidase A.

The physiological activities of several of the compounds in accordance with the present invention are described by reference to the following test examples.

### (1) Inhibition of the action of carboxypeptidase B

The inhibition of the action of carboxypeptidase B was determined by an improved version of the method for determination of the activity of angiotensin converting enzyme described in M. Hayakari et al., Analytical Biochemistry, 84, 361 - 369 (1978). The method proceeds as follows: A mixture of 0.05 mℓ of 0.05 M fibril-L-lysine (prepared by Tanpakushitsu Kenkyu Shoreikai) with 0.25 mℓ of 0.05 M Tris-HCℓ buffer solution (pH 8.0) and 0.15 mℓ of a solution containing a test compound was heated at 37°C for 3 minutes. To the heated solution, 0.05 mℓ of an aqueous solution containing 1 ug/mℓ of carboxypeptidase B purified from swine pancreas (product of C. F. Boehringer G Sohne GmbH, Mannheim) and 1 mg/mℓ of bovine albumin was added, and the mixture was subjected to reaction at 37°C for 30 minutes. The reaction was quenched by adding 0.03 mℓ of 1 N caustic soda. Fifteen minutes later, 2 mℓ of 0.05 M sodium phosphate buffer solution (pH 7.2) was added to the reaction mixture, and color reaction was performed by addition of 2 mℓ of 1% cyanuric chloride in methyl cellosolve. The mixture was left at room temperature for 15 minutes and the absorbance (a) at 382 nm was measured. The absorbance (b) of a control containing no test compound was also measured by repeating the same procedure. Blank tests were also conducted to determine the corresponding absorbances (a') and (b').

In each blank teat, the color reaction was performed by adding the enzyme solution after caustic soda so that no enzymatic reaction would take place. The percent inhibition of the action of carboxypeptidase B was calculated by the formula:

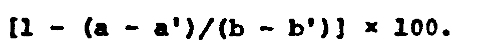

In accordance with the method shown above, compound Nos. la (L-His-C₂-L-His), 2a (L-His-C₂-L-Phe) and 6a (L-His-C2-L-Orn) exhibited 50% inhibition at concentrations of 60 µg/mℓ, 64 µg/mℓ and 112 µg/mℓ, respectively (see Table 4 below).

(2) Inhibition of the action of carboxypeptidase A The inhibition of the action of carboxypeptidase A was determined by the method described in T. Tanaka et al., The Journal of Antibiotics, 37, 682 - 684 (1984). The method proceeds as follows: A mixture of 0.05 mℓ of 0.01 M fibril-L-phenylalanine (product of Sigma Chemical Co.) with 0.25 mℓ of 0.05 M Tris-HCℓ buffer solution (pH 7.5) containing 0.9 M NaCℓ. and 0.15 mℓ of a solution containing a teat compound was heated at 37°C for 3 minutes. To the heated solution, 0.05 mℓ of a diluted aqueous solution of carboxypeptidase A purified from bovine pancreas (product of Sigma Chemical Co.) which also contained 1 mg/mℓ of purified bovine albumin was added and the mixture was enbjected to reaction at 37°C for 30 minutes. The reaction was quenched by adding 0.03 mℓ of 1 M caustic soda. Fifteen minutes later, 2 mℓ of 0.06 M sodium phosphate buffer solution (pH 7.2) was added to the reaction mixture, and color reaction was performed by addition of 2 mℓ of 1% cyanuric chloride in methyl cellosolve. The mixture was left at room temperature for 15 minutes and the absorbance (a) at 382 nm was measured. The absorbance (b) of a control containing no test compound was also measured by repeating the same procedure. Blank tests were also conducted to determine the corresponding absorbances (a') and (b'). In each blank test, the color reaction was performed by adding the enzyme solution after caustic soda so that no enzymatic reaction would take place. The percent inhibition of the action of carboxypeptidase A was calculated by the formula:

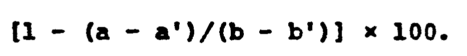

In accordance with the method shown above, compound No. 2a (L-His-C₂-L-Phe) exhibited 50t inhibition at a concentration of 8.4 µg/mℓ (see Table 4).

### (3) Inhibition of the action of angiotensin converting enzyme

The inhibition of the action of angiotensin converting enzyme was determined by an inproved version of the method for determination of the activity of angiotensin converting enzyme described in M. Hayakari et al., Analytical Biochemistry, 84, 361 - 369 (1978). The method proceeds as follows: Substrate fibril-L-histidyl-L-leucine was dissolved at a concentration of 5 mg/mt in Tris-HCℓ buffer solution (0.5 M, pH 8.0) containing 0.3 M NaCℓ. A mixture of this solution (0.05 mℓ) and 0.40 mℓ of a solution containing a test compound was heated at 37°C for 3 minutes. To the heated solution, 0.05 ml of a solution containing angiotensin converting enzyme partially purified from a bovine lung was added, and the mixture was subjected to reaction at 37°C for 30 minutes. The reaction was quenched by adding 0.03 mℓ of 1 N caustic soda. Fifteen minutes later, 2 mℓ of 0.06 M sodium phosphate buffer solution (pH 7.2) was added to the reaction mixture, and color reaction was performed by addition of 2 mℓ of It cyanuric chloride in methyl cellosolve. The mixture was left at room temperature for 15 minutes and the absorbance (a) at 382 nm was measured. The absorbance (b) of a control containing no test compound was also measured by repeating the same procedure. Blank tests were also conducted to determine the corresponding absorbances (a') and (b'). In each blank test, the color reaction was performed by adding the enzyme solution after caustic soda so that no enzymatic reaction would take place. The percent inhibition of the action of angiotensin converting enzyme was calculated by the formula:

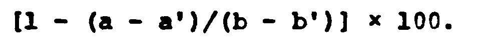

In accordance with the method shown above, it was found that compound Nos. la to 6a listed in Table 2 exhibited strong inhibition at low concentrations (see Table 4). A particularly strong inhibition was displayed by compound Nos. la (L-His-C₂-L-His) and 5a (L-His-C₂-L-AsP) had IC₅₀ (50% inhibition concentration) values of 0.7 µg/mℓ and 0.5 µg/mℓ, respectively.

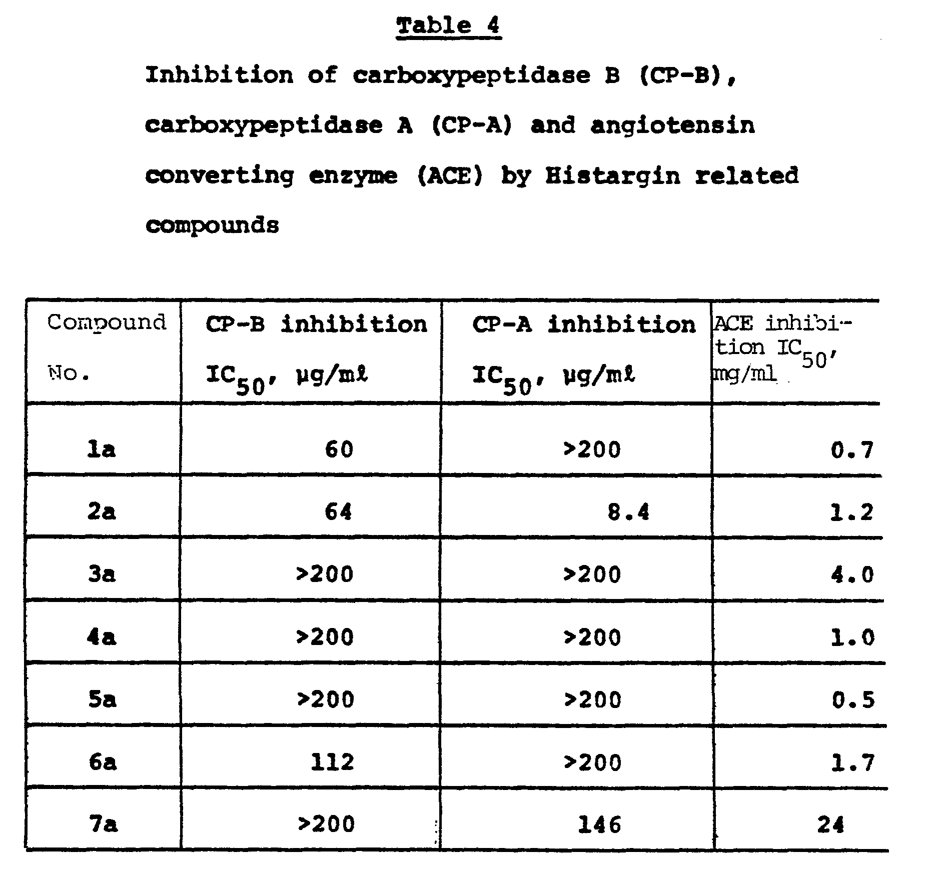

Due to the angiotensin converting enzyme (ACE) inhibiting activity the compounds of the present invention can be used as anti-hypertensive agents.

### Acute toxicity

Each of the compounds in accordance with the present invention has low toxicity and can safely be used as a medicine. The results of an acute toxicity test conducted by intraperitoneal administration of rats with compound Nos. la to 5a are summarized in Table 5.

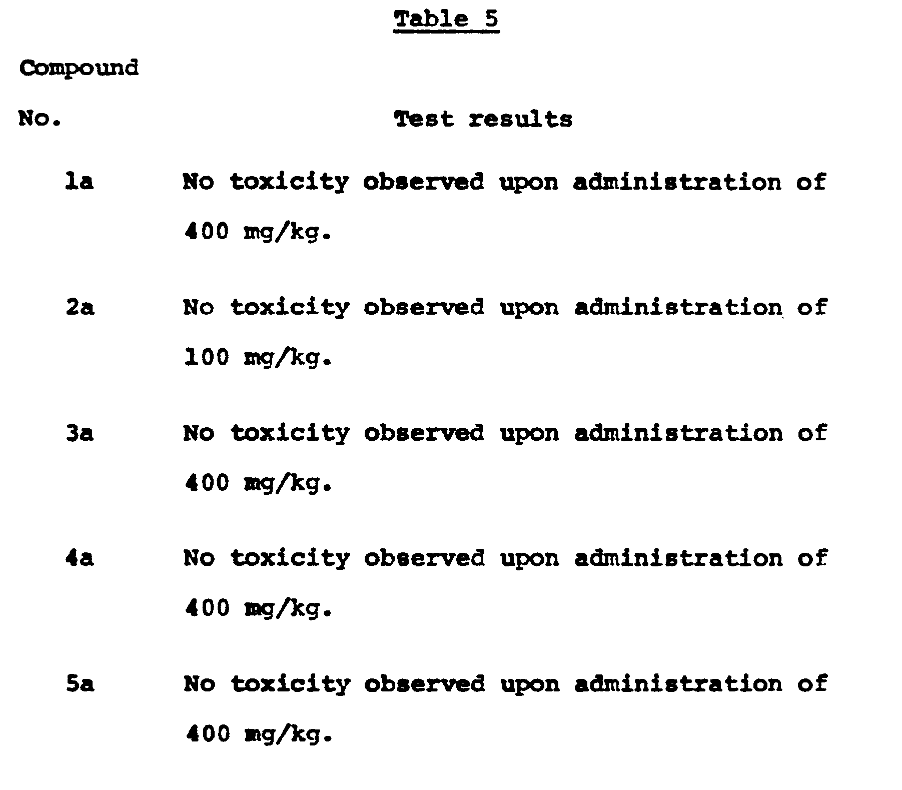

The Histargin related compounds of the present invention are novel substances and may be prepared by the following procedures.

The conventional synthetic compounds wherein the alpha-amino groups in two alpha-amino acids are bound by an ethylene group are limited to those wherein the two alpha-amino acids are identical, and such compounds have been prepared by condensing the alpha-amino acid with 1,2-dibromoethane [see Inorganic Chemistry, 7, 2405 (1968)]. No method of synthesis has been reported that uses histidine as the alpha-amino acid.

The Histargin related compounds of the present invention contain two different alpha-amino acids, and if they are reacted with 1,2-dibromoethane within the same reaction vessel, a complex product results and considerable difficulties are involved in purifying the product. The present inventors have found that these problems can be solved by performing the synthesis stepwise as shown below.

Histargin is first reacted with a 2-halogeno-1,1- dialkoxyethane of formula (II): 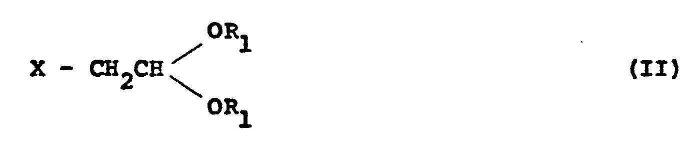 (wherein X is a halogen atom; and R₁ is a lower alkyl group) so as to form N^{a-}(2,2-dialkoxyethyl)histidine of formula (III): 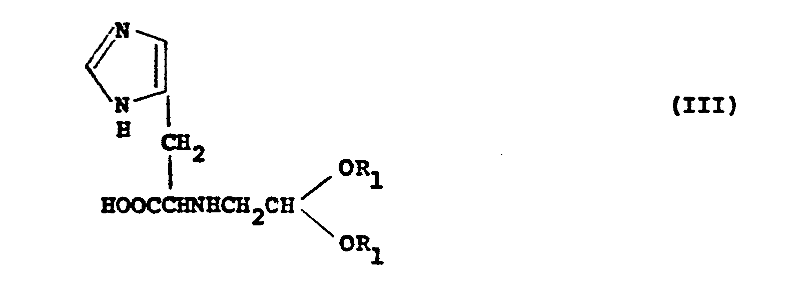 (wherein R₁ is a lower alkyl group). Compounds of formula (II) are commercially available and they include 2-bromoacetaldehyde diethyl acetal, 2-bromoacetaldehyde dimethyl acetal, 2-chloroacetaldehyde diethyl acetal and 2-chloroacetaldehyde dimethyl acetal.

Subsequently, the alpha-amino group in the histidine portion in formula (III) is protected by an amino-protecting group such as a benzyloxycarbonyl group. The aldehyde protecting group is then removed by an acid treatment so as to form an N-formylmethyl derivative of formula (IV): 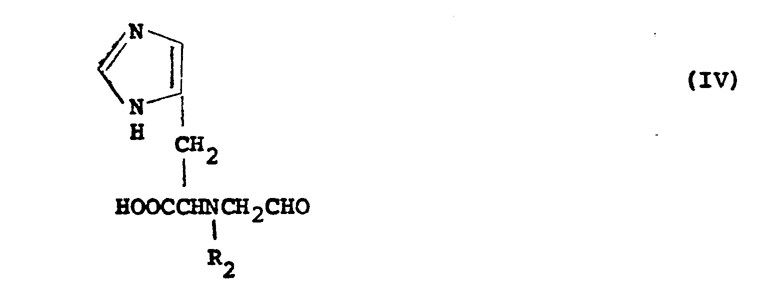 (wherein R₂ is an amino protecting group). The compound of formula (IV) has the cyclic structure represented by formula (IV'): 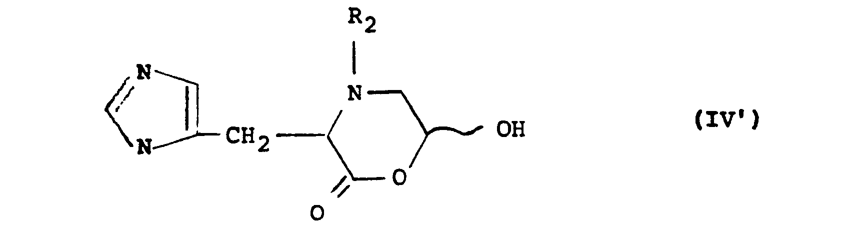 (wherein R₂ is an amino protective group). Compound (IV) is reacted, in the presence of a borohydride derivative, such as sodium cyanoborohydride, tri-tert. butoxy-borohydride, di-si-amylborane, thexylborane or the borane-THF-complex, with an alpha-amino acid which may be protected if it has a functional group in the side chain or one having protected carboxyl groups. Finally, any protective group is removed, thus providing the compound of formula (I).

Examples of the alpha-amino acid derivative that is reacted with the compound of formula (IV) for synthesizing the typical compounds listed in Table 1 include L-histidine methyl ester dihydrochloride, L-phenylalanine ethyl ester monohydrochloride, N^{ε}-benzyloxycarbonyl-L-lysine methyl ester monohydrochloride, L-proline benzyl ester monohydrochloride, and L-aspartic acid α,β-dibenzyl ester tosylate. Alpha-amino acids having unprotected carboxyl groups may also be used. In accordance with an alternative method for synthesizing the compound of formula (I), an alpha-amino acid corresponding to A in formula (I) is used as the starting material, which is condensed with the compound of formula (II), and following the conversion described above, histidine or a protected form thereof is subjected to reaction so as to obtain the desired derivative.

Among the typical compounds listed in Table 1, L-His-C₂-L-Orn indicated by compound No. 6a may also be produced by obtaining natural Histargin from the filtrate of a cultured broth of Histargin-producing microorganism of the genus Streptomyces and then by converting the cuanidyl group in that Histargin to an amino group.

The compounds synthesized by the methods shown above may be esterified by conventional techniques so as to obtain compounds wherein all the carboxyl groups present are esterified. The intermediates for the synthesis shown above may have part of the carboxyl groups protected by an ester; in this case, Histargin related compounds having part of the carboxyl groups esterified may be obtained by leaving the ester intact and removing any other protective groups.

### Examples

The following are provided as further illustrations of the invented compound and are not to be construed as limiting.

### Example 1-1

### Synthesis of N^{α}-(2,2-diethoxyethyl)-L-histidine

Fifty grams (0.32 mol) of L-histidine and 44.5 g of potassium carbonate (anhydrous) were dissolved in a mixture of ethanol (500 mt) and water (500 mt). To the solution, 63.5 g (0.32 mol) of bromoacetaldehyde diethyl acetal was added and the mixture was heated under reflux for a day. After cooling, the mixture was diluted with 1,000 mt of water, adjusted to a pH of 6.8, and had the ethanol distilled off under vacuum. The precipitate of unreacted L-histidine was filtered off, and the aqueous layer was washed twice with ethyl acetate, followed by concentration to 500 mℓ under vacuum. To the concentrated aqueous layer, 29.2 g of sodium chloride (NaCt) was added to form a 0.5 M NaCt solution. This solution was passed through a column that had been filled with 2,000 mℓ of Diaion^{®}HP-20 (product of Mitsubishi Chemical Industries Limited) preconditioned with 0.5 M NaCℓ. After elution with 6,000 mℓ of 0.5 M NaCℓ, 15,000 mℓ of water and 7,000 mℓ of 20% methanol, the fractions containing the end compound were collected and evaporated to dryness under vacuum. Ethanol (150 mℓ) was added to the residual solid and the insoluble NaCℓ was filtered off. The above procedure was repeated twice, and the obtained filtrate was crystallized with ethyl ethanol, thereby obtaining 31.8 g of N^{α}-(2,2-diethoxyetllyl)-L-histidine. Yield: 36.3t; m.p. 160 - 162°C.;

[α]²⁵_{D} -27.4° (c = 1, methanol;

IR(KBr) ν (cm⁻¹) = 2970, 2870, 1645, 1580, 1570, 1450, 1380, 1300, 1125, 1090, 1065, 845, 800, 630, 550, 480;

NMR (d-MeOH) δ = 1.20 (t, CH₃ x 2, T = 7 Hz), 3.0 ~ 3.5 (m, CH₂ x 2), 3.5 ~ 4.0 (m, 5 H), 4.79 (t, CH, T = 5 Hz), 6.98 (s, CH), 7.62 (s, CH).

### Example 1-2

### Synthesis of N^{α}-bensyloxycarbonyl-N^{α}-(2,2-diethoxyethyl)-L-histidine

19.5 g (71.7 nmol) of N^{α}- (2, 2-diethoxyethyl) -L-histidine was dissolved in 71.7 mℓ of 1 N NaOH. With vigorous stirring under cooling with ice, 22.6 mℓ of benzyoxycarbonyl chloride was added in five portions at 10-minute intervals. Throughout this addition, the pH of the reaction mixture was held at between 9.0 and 10.0 with 2 N NaOH. Following the addition of the reagent, the mixture was stirred for another 2 hours. Three extractions were conducted with ethyl acetate (150 mℓ), and the combined ethyl acetate layers were washed twice, each time with 1 N HCℓ and saturated NaCℓ, followed by drying with anhydrous sodium sulfate. By distilling off the solvent, 40.1 g of _{N}^{a},_{N}^{im-} dibenzyloxycarbonyl-N^{α}-(2,2-diethoxyethyl)-L-histidine was obtained; this product was subsequently dissolved in 250 ml of dry methanol and mixed under agitation with 27.7 mℓ of a methanol solution of 28% sodium methylate for 2.5 hrs at room temperature. The reaction mixture was diluted with 250 mℓ of water, adjusted to a pH of 7.0 and had the methanol distilled off under vacuum. The resulting aqueous layer was washed twice with ethyl acetate. The aqueous layer was evaporated to dryness under vacuum, and after adding a small amount of methanol to the residue, the insoluble NaCℓ. was filtered off. The procedures shown above were repeated twice, and the resulting filtrate was concentrated under vacuum to obtain 26.0 g of syrupy N^{α}-benzyloxycarbonyl-N^{α}-(2,2-diethoxyethyl)-L-histidine. Yield: 89.38; [α]²⁵_{D} - 58. 9° (c = 1, MeOH);

IR(KBr) v (cm⁻¹) = 3380, 2960, 1680, 1600, 1460, 1450, 1390, 1340, 1250, 1125, 1050, 1000, 810, 760. 690;

NMR (d-MeOH) 6 - 1.05 (t, CH₃ x 2, T - 6 Hz), 3.0 ~ 4.0 (m, 9 H), 4.5 (m, 1 H), 5.10 (s, CH₂), 6.70 (₈, CH), 7.32 (s, 5 H), 7.60 (s, CH).

### Example 1 - 3

### Synthesis of N^{α}-benzyloxycarbonyl-N^{α}-(formylmethyl)-L-histidine

25.9 g (63.9 mmol) of N^{α}-benzyloxycarbonyl-N^{α}-(2,2-diethoxyethyl)-L-histidine was dissolved in a mixture of ethanol (60 mℓ) and water (140 mℓ). To the solution, 1 N HCℓ was added and the mixture was stirred for 4 hrs at room temperature. Thereafter, the reaction mixture was adjusted to a pH of 7.0 and the ethanol was distilled off under vacuum. The aqueous layer was passed through a column packed with 4,000 mℓ of Diaion®HP-20. After rinsing with 4,000 mℓ of water, the column was eluted with 25% methanol. The fractions containing the end compound were collected and concentrated under vacuum to obtain 15.1 g of a pale yellow powder of N^{α}-benzyloxycarbonyl-N^{α}-(formylmethyl)-L-histidine. Yield: 71.5%;

[α]²⁵_{D} - 13.1° (c - 1, MeOH);

I_{R}(KBr) v (cm⁻¹) = 3450, 2950, 1700, 1600, 1420, 1370, 1310, 1260, 1220, 1190, 1130, 1110, 990, 825, 785, 740, 705, 670;

NMR (d-MeOH) 6 - 3.1 ~ 3.4 (2 H), 3.9 ~ 4.2 (m, 2 H), 4.3 ~4.7 (m, 1 H), 5.09 (s, CH₂), 5.6 ~ 5.9 (m, 1 H), 6.70 (s, 1 H), 7.28 and 7.32 (each s, 5 H), 7.65 (s, 1 H).

### Example 1 - 4

### Synthesis of L-His-C₂-L-His (compound No. la)

A methanol (20 mℓ,) solution of 1.72 g (5.2 mmol) of N^{α}-benzylozycarbonyl-N^{α}-(formylmethyl) -L-histidine was mixed with a solution that comprised 2.52 g (10.4 mmol) of L-histidine methyl ester dihydrochloride in 10 mℓ of water and which had been adjusted to a pH of 6.7. With stirring under cooling with ice, 327 mg (5.2 mmol) of sodium cyanoborohydride was added in small portions. Thereafter, the reaction mixture was stirred for an additional 4 hours at room temperature and concentrated under vacuum. The resulting residue was dissolved in 10 mℓ of water and passed through a column packed with 500 mℓ of Diaion® HP-20.

After rinsing with 1,500 mℓ of water, the column was eluted with 3,000 mℓ of 25% methanol and 3,000 mℓ of 50% methanol. The fractions containing the end compound were collected and concentrated under vacuum to obtain 2.29 g of Z-L-His-C₂-L-His-Ome. This compound had characteristic signals at 6 3.69 (s, CH₃), 5.03 (s, CH₂) and 7.28 (s, 5H) in the NMR spectrum (d-MeOH). This compound was dissolved in methanol (40 mℓ) and mixed under agitation with 13 mℓ of 1 N NaOH for 3.5 hrs at room temperature. Thereafter, the reaction mixture was adjusted to a pH of 6.8 and had the methanol distilled off under vacuum. The resulting aqueous layer was passed through a column packed with 350 mℓ of Diaion®HP-20. Following rinsing with 1,000 mℓ of water, the column was eluted with 25% methanol. The fractions containing the end compound were concentrated under vacuum to obtain 1.12 g of 2-L-His-C₂-L-His (total yield: 45.8%). This compound had characteristic signals at 6 2.6 - 3.5 (m, CH₂ ^{x} 4), 4.2 - 4.6 (m, CH ^{x} 4), 5.00 (s, CH₂), 6.56 (s, CH), 6.80 (s, CH), 7.23 (s, 5H) and 7.45 (s, CH x 2) in the NMR spectrum (d-MeOH).

A portion (465 mg) of the Z-L-His-C₂-L-His was dissolved in a mixture of ethanol (5 mℓ) and water (5 mℓ). To the resulting mixture, 3 mℓ of 1 N HCf and 25 mg of 10% Pd-carbon were added, followed by stirring for 3.5 hours under a hydrogen stream. The catalyst was filtered off and the filtrate was passed through a column packed with 50 mℓ of Dowex®50W× 8 (H type, product of Dow Chemical Co.), and following rinsing with 200 mℓ of water, the fractions containing the end compound were collected and concentrated under vacuum. The residue was crystallized from ethanol to obtain 225 mg of the end compound, L-His-C₂-L-His. Yield: 67.0%, m.p. 227 - 232°C (with decompn). [α]²⁵_{D} + 17.1° (c - 1, 6 N HCℓ).

### Example 2

### Synthesis of L-His-C₂-L-Phe (compound No. 2a)

Reaction was performed as in Example 1 - 4 by using 910 mg (2.8 mmol) of N^{α}-benzyloxycarbonyl-N^{α}-(formylmethyl)-L-histidine, 1.263 a (5.5 mmol) of L-phenylalanine ethyl ester monohydrochloride and 173 mg (2.8 mmol) of sodim borohydride. The reaction mixture was purified on a column packed with 250 mℓ of Diaion® HP-20, thereby obtaining 1.39 g of Z-L-His-C₂-L-Phe-OEt. This compound had characteristic signals at δ 1.17 (t, CH₃, T = 7 Hz), 4.10 (q, CH₂, T - 7 Hz), 5.00 (s, CH₂), 7.20 (s, 5H) and 7.27 (s, 5H) in the NMR spectrum (d-MeOH) . This compound was hydrolyzed as in Example 1-4 and purified on a column packed with 250 mℓ of Diaion^{®} HP-20, thereby obtaining 773 mg of z-L-His-C₂-L-Phe (total yield: 58.5%) .

A portion (716 mg) of z-L-His-C₂-L-Phe, 4.5 mℓ of 1 N BCl and 50 mg of 10% Pd-carbon were subjected to the same reaction as in Example 1 - 4, and the reaction product was purified on a column packed with 50 mℓ of Dowex^{®}50 _{W} x 8 (B type). Crystallization from ethanol produced 277 mg of the end compound, L-His-C₂-L-Phe. Yield: 53.7t. m.p. 248 - 250°C (with decompn). [α]²⁵_{D}+ 38.1° (c - 1, 6 N HCℓ).

### Example 3

### Synthesis of L-His-C₂-L-Lys (compound No. 3a)

Reaction was performed as in Example 1 - 4 by using 1.71 g (5.2 mmol) of N^{α}-benzyloxycarbonyl-N^{α}-(formylmethyl)-L-histidine, 2.30 g (6.9 mmol) of N^{α}-bensyloxycyarbonyl-L-lysine methyl ester monohydrochloride and 324 mg (5.2 mmol) of sodium cyanoborohydride. The reaction mixture was purified on a column packed with 500 mℓ of Diaion® HP-20. From the eluate with 75% methanol, 3.27 g of a residue comprising z-L-His-C₂-L-Lys (Z)-OMe was obtained. This compound had characteristic signals at δ ₃.72 (a, CH₃), 5.02 (S, C_{H2} x 2) and 7.29 (s, 10H) in the MMR spectrum (d-MeOH).

This compound was hydrolyzed as in Example 1 - 4 and purified on a column packed with 500 mℓ of Diaion^{R} _{H}P-20. From the eluate with 75% methanol, 1.93 g of Z-L-His-C₂-L-Lys (Z) was obtained (total yield: 62.6%). This compound had characteristic signals at δ 1.2 - 2.1 (m, CH₂ x 3), 2.8 - 3.6 (a, CH₂ x 5), 4.2 - 4.7 (m, CH x 2), 5.04 (s, CH₂), 5.07 (s, CH₂), 6.85 (a, CH), 7.30 (s, 5H) and 8.03 (s, CH) in the NMR spectrum (d-MeOH).

A portion (1.88 g) of the Z-L-His-C₂-L-Lys(Z), 9.6 mℓ of 1 N HCℓ and 100 mg of 10% Pd-carbon were subjected to the same reaction as in Example 1 - 4. After filtering off the catalyst, the filtrate was concentrated under vacuum and the residue was dissolved in 10 mℓ of water. The solution was passed through a column packed with 150 mℓ of CM-Cephadex® C-25 (Na type, product of Pharmacia Labs., Inc.), gradient elution with 1,000 mℓ of water and 1,000 mℓ of 1 M NaCℓ was performed. The fractions containing the end compound were collected, and the combined fractions were passed through a column packed with 50 mℓ of Dowex® 50 W x 8 (H type), and following rinsing with water, the column was eluted with 2 N NH₄0H. The fractions containing the end compound were collected and concentrated under vacuum. The resulting residue (powder) was dissolved in 2 ml of water and freeze-dried to produce 219 mg of L-His-C₂-L-Lys as a white powder. Yield: 23.1%; [α]²⁵_{D} - 32.0° (c - 1, 6N HCℓ).

### Example 4

### Synthesis of L-His-C₂-L-Pro (compound No. 4a)

Reaction was performed as in Example 1 - 4 by using 4.73 g (14.3 mol) of N^{α}-benzyloxycarbonyl-N^{α}-(formylmethyl)-L-histidine, 6.88 g (28.6 mmol) of L-proline benzyl ester monohydrochloride and 899 mg (14.3 mmol) of sodium cyanoborohydride. The reaction product was purified on a column packed with 1,000 mℓ of Diaion® HP-20, and from the eluate with 75%, 2.21 g of Z-L-His-C₂-L-Pro·OBzℓ was obtained. This compound had characteristic signals at 6 1.7 - 2.4 (a, CH₂ x 2), 2.8 - 3.8 (m, CH₂ x 4), 4.2 - 4.7 (m, CH x 2), 5.00 (s, CH₂), 5.10 (s, CH₂), 6.72 (s, CH), 7.22 (s, SH), 7.27 (s, 5H) and 7.80 (s, CH) in the NMR spectrum (d-MeOH). Using 2.10 g of Z-L-His-C₂-L-Pro·OBzℓ, 12 mℓ of 1 N HCℓ and 100 mg of 10% Pd-carbon, a subsequent reaction was performed as in Example 1 - 4. The catalyst was filtered off and the filtrate was concentrated under vacuum, and the residue was dissolved in 10 mℓ of water. The solution was passed through a column packed with 300 at of CM-Cephadex® C-25 (Na type) and the column was eluted with water. The fractions containing the end compound were collected and passed through a column packed with 100 mℓ of Dowex® 50 w x 8 (H type). Following rinsing with water, the column was eluted with 2 N NH₄OH. The fractions containing the end compound were collected and concentrated under vacuum. The resulting residue was dissolved in 5 mℓ of water, and the solution was freeze-dried to give 646 mg of L-His-C₂-L-Pro as a white powder. Total yield: 15.8%.

[α]²⁵_{D} - 24.2° (c - 1, 6 N HCℓ).

### Example 5

### Synthesis of L-His-C₂-L-Asp (compound No. 5a)

Reaction was conducted as in Example 1 - 4 using 2.00 g (6.1 mmol) of N^{α}-benzyloxycarbonyl-N^{α}-(formylmethyl)-L-histidine, 1.61 g (12.1 mmol) of L-aspartic acid and 380 mg (6.1 mmol) of sodium cyanoborohydride. The reaction mixture was passed throngh a column packed with 250 at of Diaion® HP-20 that had been conditioned with 0.5 M NaCℓ. After rinsing with 1,000 mℓ of 0.5 M NaCℓ, the column was eluted with water. The fractions containing the end compound were collected and concentrated under vacuum to obtain 292 mg of Z-L-His-C₂-L-Asp.

Using 292 mg of the Z-L-His-C₂-L-Asp, 2 mℓ of 1 N HCℓ and 20 mg of 10% Pd-carbon, a subsequent reaction was performed as in Example 1-4. The filtrate was passed through a column packed with 100 ml of CM-Cephadex® C-25 (Na type) and the column eluted with water. The fraction containing the end compound were collected and concentrated under vacuum. The resulting residue was subjected to preparatory thin-layer chromatography on silica gel 60 F₂₅₄ that was produced by Merck Co. and had a thickness of 2 mm. After development with ethanol:C-NH₄OH (1:1), the band containing the end compound (Rf 0.67) was scraped and eluted with ethanol:C-NH₄0H (1:1). The eluate was concentrated under vaccum and the resulting residue was passed through a column packed with 20 ml of Dowex® 50 W x 8 (H type). After rinsing with water, the column was eluted with 2 N NH₄0H. The fractions containing the end compound were collected and concentrated under vacuum. The resulting residue was crystallized from methanol to obtain 10.7 mg of L-His-C₂-L-Asp. m.p. 250-252°C (with decompn).

[α]²⁵_{D} + 39.9° (c = 1, 6 N HCℓ).

### Example 6

### Synthesis of L-His-C₂-L-Asp (compound No. Sa) by alternative route

Reaction was performed as in Example 1 - 4 using 3.00 g (9.1 mmol) of N^{α}-benzyloxycarbonyl-N^{α}-(formylmethyl)-L-histidine, 8.79 g (18.1 mmol) of L-aspartic acid α,β-dibenzyl ester tosylate and 569 mg (9.1 mmol) of sodium borohydride. The reaction mixture was concentrated under vacuum and the resulting residue was dissolved in 20 mℓ of water and washed twice with ethyl acetate. The aqueous layer was concentrated under vacuum to half its volume and passed through a column packed with 200 mℓ of Diaion® MP-20. After rinsing with 300 mℓ of water and 300 mℓ of 50% methanol, the column was eluted with methanol. The fractions containing the end compound were collected and concentrated under vacuum to obtain 2.52 g of Z-L-His-C₂-L-Asp(OBzℓ)-OBzℓ.

Using 2.34 g of Z-L-His-C₂-L-Asp(OBzℓ)-OBzℓ, 10 mℓ of 1 N HCℓ and 200 mg of 10% Pd-carbon, a subsequent reaction was conducted as in Example 1 - 4. Thereafter, the catalyst was filtered off and the filtrate was concentrated under vacuum. The resulting residue was dissolved in 10 mℓ of water and the solution was passed through a column packed with 250 mℓ of Diaion® HP-20 and the column was eluted with water. The fractions containing the end compound were collected and concentrated under vacuum. The resulting residue was crystallised from methanol to obtain 314 mg of L-His-C₂-L-Asp. Total yield: 11.8%.

### Example 7

### Synthesis of L-His-C₂-L-Orn (compound No. 6a)

Histargin (268 mg) obtained from the filtrate of a cultured broth was dissolved in 40 mℓ of saturated aqueous barium hydroxide, and the solution was heated under reflux in an oil bath (105°C) for 2 hrs. After cooling, carbon dioxide was blown into the reaction mixture and the resulting precipitate was filtered off. The filtrate was passed through a column packed with 45 mℓ of Dowex® 50 W × 4 (H type). After rinsing with water, the column was eluted with 25% NH₄OH. The fractions containing the end compound were collected and evaporated to dryness under vacuum, thereby producing a crude powder (224 mg). This powder was dissolved in 10 mℓ of 1 M pyridine-acetic acid buffer solution (pH 5.0). The resulting solution was passed through a column packed with 80 mℓ of Dowex® 50 W x 4 (Py type) that had been equilibrated with the same buffer. After elution with the same buffer, the fractions containing the end compound were collected and adsorbed on 80 mℓ of Dowex® 50 W × 4 (H type). After rinsing with water, the column was eluted with 25% NH₄OH. The fractions containing the end compound were collected and concentrated under vacuum. The resulting residue was dissolved in 2 mℓ of water and freeze-dried to give 91.5 mg of L-His-C₂-L-Orn as a white powder.

[α]²⁵_{D} + 30.8° (c = 1, 6 N HCℓ).

### Example 8

### Synthesis of L-His-C₂-L-Phe-OEt (compound No. 7a)

A portion (301 mg) of the Z-L-His-C₂-L-Phe-OEt obtained in Example 2 was dissolved in 10 ml of a mixture of ethanol and water (1:1). To the resulting solution, 1.7 ml of 1 N HCl and 25 mg of 10% Pd-carbon were added and the mixture was agitated in a hydrogen stream for 4 hrs. Thereafter, the catalyst was filtered off and the filtrate was neutralised and concentrated under vacuum to half its volume. The filtrate was then passed through a column packed with 300 mℓ of CM-Cephadex® C-25 (Na type) and the column was eluted with water. The fractions containing the end compound were collected and concentrated under vacuum. The resulting residue was dissolved in 1 mℓ of methanol and the solution was passed through a column packed with 100 mℓ of Cephadex® LH-20 (Pharmacia Labs., Inc.). After elution with methanol, the fractions containing the end compound were collected and concentrated under vacuum. The resulting residue was dissolved in 1 mℓ of water and freeze-dried to give 60.5 mg of a monohydrochloride of L-His-C₂-L-Phe-OEt as a white powder

[α]²⁵_{D} + 33.4° (c = 1, 6 N HCℓ).

### Example 9

### Synthesis of L-His-OMe-C₂-L-His-OMe (compound No. 8a)

A portion (1.05 g) of the Z-L-His-C₂-L-His-OMe obtained in Example 1 - 4 was dissolved in 10 mℓ of methanol and methylated by adding a diazomethane ether solution under cooling with ice. After distilling off the solvent, the residue was dissolved in 15 mℓ of an ethanol-water (1 : 1) mixture. To the resulting solution, 5 mℓ of 1 N HCℓ and 150 mℓ of 10% Pd-carbon were added and the mixture was stirred for 3.5 hrs in a hydrogen stream. The catalyst was filtered off and the filtrate was neutralized. Thereafter, the filtrate was concentrated under vacuum to half ita volume and passed through a column packed with 200 mℓ of CM-Cephadex® C-25 (Na type). The column was subjected to gradient elution with 1,500 mℓ of water and an equal volume of 1 M NaCℓ. The fractions containing the end compound were collected and concentrated under vacuum. The resulting residue was dissolved in 5 mℓ of methanol and the insoluble NaCℓ was filtered off. These procedures were repeated twice. The residue was dissolved in 2 mℓ of methanol and the solution was passed through a column packed with 150 mℓ of Cephadex® LH-20, followed by elution with methanol. The fractions containing the end compound were collected and concentrated under vacuum. The resulting residue was dissolved in 2 mℓ of water and freeze-dried to obtain 176 mg of an L-His-OMe-C₂-L-His-OMe dihydrochloride as a white powder.

[α]²⁵_{D} + 15.3° (c = 1, 6 N HCℓ).

### Advantages of the Invention

As will be apparent from the foregoing description, the present invention provides a Histargin related compound that exhibits a great ability to inhibit the action of angiotensin transferase and which hence is useful as a hypotensive.

## Claims

1. A compound of the formula I 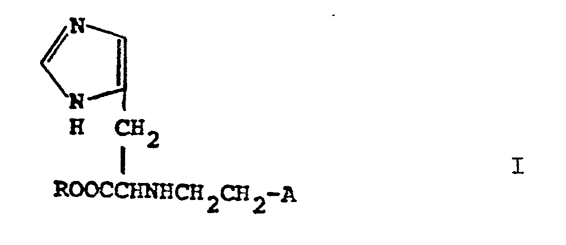 wherein A is a residual group obtained by removing one hydrogen atom from the a-amino group in an a-amino acid (excluding arginine) or a lower alkyl ester thereof; R is a hydrogen atom or a lower alkoxy group, or a salt thereof.

2. A compound as claimed in claim 1 wherein A is a residual group obtained by removing one hydrogen atom from the following a-amino acids:
glycine, alanine, alpha-aminobutyric acid, proline, valine, norvaline, isoleucine, alloisoleucine, leucine, norleucine, serine, homoserine, threonine, allothreonine, o-methylserine, o-ethylserine, o-methylhomoserine, o-ethylhomo- serine, o-methylthreonine, o-ethylthreonine, o-methylallo- threonine, o-ethylallothreonine, ornithine, lysine, aspartic acid, glutamic acid, asparagine, glutamine, phenylalanine, tyrosine, histidine, tryptophane, cysteine, homocysteine, s-methylcysteine, s-ethylcysteine, methionine or ethionine.

3. A compound as claimed in claim 2 wherein the a-amino residue may be any of the L-, D- or DL-type.

4. A process for the preparation of a compound of the formula I 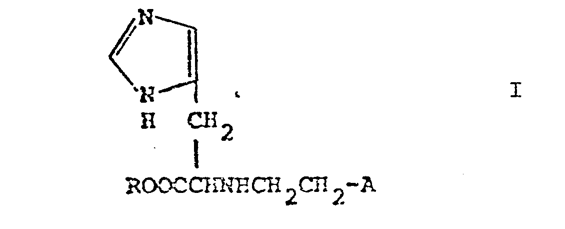 wherein A is a residual group obtained by removing one hydrogen atom from the alpha-amino group in an alpha-amino acid (excluding arginine) or a lower alkyl ester thereof; R is a hydrogen atom or a lower alkyl group, or a salt thereof, which comprises reacting a compound of the formula 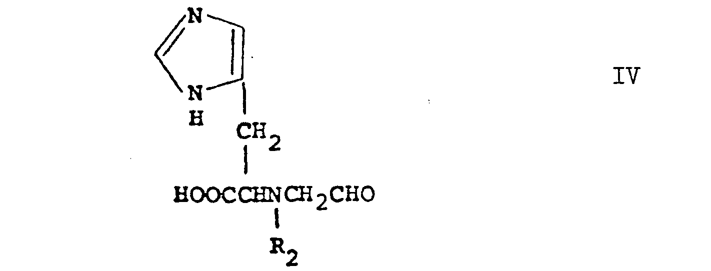 or 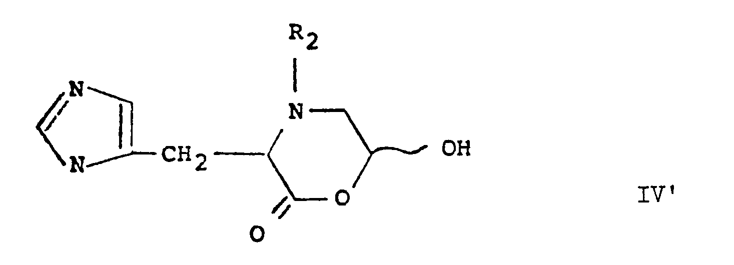 with an a-amino acid of the formula HA, wherein A is as defined above, in the presence of a borohydride derivative, and optionally removing protective groups in a conventional manner.

5. The process as claimed in claim 4 wherein sodium cyanoborohydride is used as the borohydride derivative.

6. A pharmaceutical composition comprising as the active ingredient a compound of the formula I as defined in claim 1 or a physiologically acceptable salt thereof in association with a pharmaceutically acceptable carrier.

7. Use of a compound as claimed in claim 1 or a physiologically acceptable salt thereof for the preparation of a medicament having antihypertensive activity.

## Claims (Claims for the following Contracting State(s) : Austria)

1. A process for the preparation of a compound of the formula I 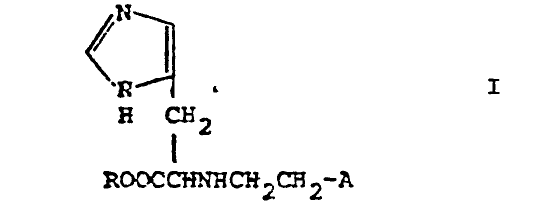 wherein A is a residual group obtained by removing one hydrogen atom from the alpha-amino group in an alpha-amino acid (excluding arginine) or a lower alkyl ester thereof; R is a hydrogen atom or a lower alkyl group, or a salt thereof, which comprises reacting a compound of the formula 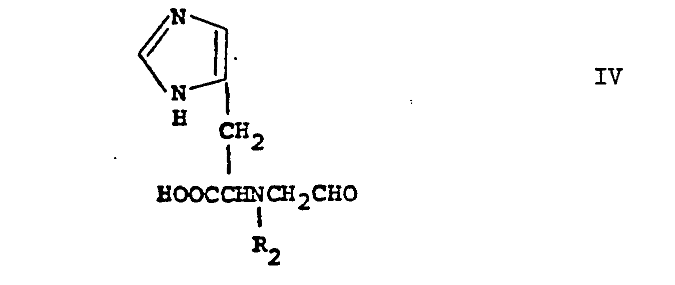 or 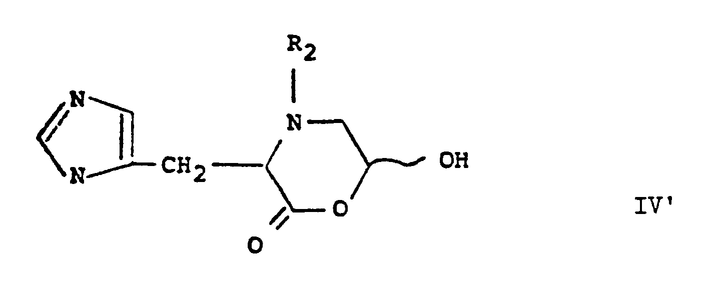 with an a-amino acid of the formula HA, wherein A is as defined above, in the presence of a borohydride derivative, and optionally removing protective groups in a conventional manner.

2. The process as claimed in claim 1 wherein A is a residual group obtained by removing one hydrogen atom from the following a-amino acids:
glycine, alanine, alpha-aminobutyric acid, proline, valine, norvaline, isoleucine, alloisoleucine, leucine, norleucine, serine, homoserine, threonine, allothreonine, o-methylserine, o-ethylserine, o-methylhonoserine, o-ethylhomo- serine, o-methylthreonine, o-ethylthreonine, o-methylallo- threonine, o-ethylallothreonine, ornithine, lysine, aspartic acid, glutamic acid, asparagine, glutamine, phenylalanine, tyrosine, histidine, tryptophane, cysteine, homocysteine, s-methylcysteine, s-ethylcysteine, methionine or ethionine.

3. The process as claimed in claim 2 wherein the a-amino residue may be any of the L-, D- or DL-type.

4. The process as claimed in claim 1 wherein sodium cyanoborohydride is used as the borohydride derivative.
